# EUROPÄISCHE PATENTSCHRIFT

(11) **EP 3 446 625 B1**
(45) Veröffentlichungstag und Bekanntmachung des Hinweises auf die Patenterteilung: **26.01.2022**
(21) Anmeldenummer: 17187957.0
(22) Anmeldetag: 25.08.2017
(51) Int. Cl.: A61B 5/00, A61B 5/20

(54) **VERWENDUNG EINER SIGNALVORRICHTUNG ZUR ERMITTLUNG EINES KATHETERISIERUNGSBEDARFS**
USE OF A SIGNALLING DEVICE FOR THE DETERMINATION OF A CATHETERISATION REQUIREMENT
UTILISATION D'UN DISPOSITIF DE SIGNAL POUR DETERMINER UNE DEMANDE DE CATHÉTÉRISME

(43) Veröffentlichungstag der Anmeldung: 27.02.2019
(73) Patentinhaber: Perlitz, Volker, 52074 Aachen (DE)
(72) Erfinder: Perlitz, Volker, 52074 Aachen (DE)
(74) Vertreter: Bauer, Dirk

(56) Entgegenhaltungen:
- WO-A1-2017/017426
- US-A1- 2012 136 231
- US-A1- 2014 228 649

## Beschreibung

Die Erfindung betrifft die Verwendung einer Signalvorrichtung zur Ermittlung eines individuellen Katheterisierungsbedarfs einer Person vor Entstehen eines Blasenhochdrucks.

Ein durch Blasenhochdruck entstehender Schmerz-Stressreiz wird von neurogen geschädigten Personen (z.B. mit Querschnittslähmung, Multipler Sklerose oder Halbseitenlähmung nach apoplektischem Insult) nicht unmittelbar wahrgenommen. Ein solcher Schmerz-Stressreiz verbindet sich individuell unterschiedlich neben motorischer Unruhe mit vegetativen Reaktionen, wie erhöhtem Blutdruck, Zunahme von Herzfrequenz, Atemfrequenz, Hautdurchblutung und elektrischer Hautleitfähigkeit.

Neben der medikamentösen Therapie mit Anticholinergika und operativen Maßnahmen wie der Durchtrennung des Musculus sphincter vesicae externus (Reynard JM, Vass J, Sullivan ME, Mamas M: Sphincterotomy and the treatment of detrusor-sphincter dyssynergia: current status, future prospects. Spinal Cord (2003) 41, 1-11. doi:10.1038/sj.sc.3101378) wird der intermittierende Katheterismus angewendet, um eine Überfüllung der Blase zu vermeiden: Hierbei wird die Blase in etwa gleichen Intervallen von circa 6 Stunden über einen Blasenkatheter entleert.

Da weder die Harnproduktion noch die Kapazität der Blase konstant sind - beispielsweise können Infektionen oder psychophysische Belastungen die Kapazität reduzieren, kommt es aufgrund der festen Intervalle regelmäßig zu Überversorgung, wenn die Blase noch nicht ausreichend oder zu Unterversorgung, wenn sie bereits zu sehr gefüllt ist. Bei Unterversorgung können Sekundärschäden bis hin zum Nierenversagen auftreten, Überversorgung ist zu vermeiden, weil bei jedem Einführen eines Katheters als invasivem Vorgang immer das Risiko einer Keimeinschleppung mit Infektion oder einer Verletzung der Harnröhre besteht (Frankel HL et al.: Long-term survival in spinal cord injury: a fifty year investigation. Spinal Cord 1998; 36: 868-869).

Um den individuellen Katheterisierungsbedarf vor Entstehen eines Blasenhochdrucks zu erkennen, ist es allgemein bekannt, den Füllungszustand der Blase nicht-invasiv durch sonographische oder impedanzvolumetrische Messungen (Schlebusch T: Impedanz-Zystovolumetrie. Aachen, Techn. Hochsch.,

Diss., 2015) abzuschätzen. WO 2017/017426 offenbart eine solche Vorrichtung zur sonographischen Bestimmung des Füllzustands der Blase.

### Aufgabe

Der Erfindung liegt die Aufgabe zugrunde, den geeignetsten Zeitpunkt zur Katheterisierung zu erkennen, ehe Blasenhochdruck entsteht.

### Lösung

Gemäß der Erfindung wird die Verwendung einer Signalvorrichtung zur Ermittlung eines individuellen Katheterisierungsbedarfs einer Person vor Entstehen eines Blasenhochdrucks vorgeschlagen, wobei die Signalvorrichtung ein Datenverarbeitungsgerät aufweist und Sensoren für Herzfrequenz, Atmung und Bewegung einer Person, die jeweils Funkverbindungsmittel zur drahtlosen Verbindung mit dem Datenverarbeitungsgerät aufweisen, wobei das Datenverarbeitungsgerät dazu eingerichtet ist, von den Sensoren aufgenommene Daten zu einem physiologischen Zustand der Person drahtlos zu empfangen und bei einer zunehmenden (progressiven) und/oder sprunghaften Änderung des Zustands ein akustisches, visuelles und/oder taktiles Signal zu erzeugen und wobei die Signalvorrichtung ein Kontrollmittel zum manuellen Validieren des Signals aufweist.

Als Datenverarbeitungsgerät kommt insbesondere ein bildschirmloser Kleinstcomputer, typischer Weise mit eigener Spannungsversorgung zum Einsatz, der fest an einem Bett oder einem Krankenfahrstuhl befestigt oder in einer Tasche mitgeführt werden kann. Alternativ kommt als Datenverarbeitungsgerät auch ein Smartphone der Person in Betracht.

Zur Messung der Herzfrequenz kommen als Sensor insbesondere herkömmliche EKG-Sensoren, zur Messung der Atmung Sensormatten oder Bewegungssensoren, zur Messung unwillkürlicher Bewegungen von Rumpf und/oder auch von einer Lähmung betroffenen Gliedmaßen, gleichfalls Bewegungssensoren zum Einsatz.

Die Funkverbindung jedes einzelnen Sensors zum Datenverarbeitungsgerät erlaubt einerseits eine freie Positionierung der Sensoren an der Person oder in deren Kleidung, zum Zweiten werden störanfällige und als lästig empfundene Kabelverbindungen vermieden und schließlich können die in vielen Kleincomputern und mobilen Geräten standardmäßig vorhandenen Möglichkeiten für Funkverbindungen, insbesondere Bluetooth, DECT und NFC genutzt werden.

In der erfindungsgemäßen Signalvorrichtung kann das Datenverarbeitungsgerät insbesondere durch eine Software dazu eingerichtet sein, von den Sensoren aufgenommene Daten zu einem Zustand der Person drahtlos zu empfangen und bei einer zunehmenden (progressiven) und/oder sprunghaften Änderung des Zustands ein akustisches oder taktiles Signal zu erzeugen. Die softwaretechnische Einrichtung erleichtert die Anpassung des Datenverarbeitungsgeräts an individuell unterschiedliche oder sich ändernde Anforderungen und Sensorkonfigurationen, aber auch im Fehlerfall die Wartung und Reparatur, insbesondere durch Aktualisierung der Software.

Das Erkennen einer zunehmenden (progressiven) und/oder sprunghaften Änderung bedingt mindestens die vorübergehende Speicherung von Messwerten und eine Kenntnis des zeitlichen Abstands zu den nächsten Messwerten.

Der Erfindung liegt die Erkenntnis zugrunde, dass die vegetativen Schmerz-Stressreaktionen bei Blasenhochdruck zwar individuell bei jedem Patienten verschieden, aber gleichwohl immer mit einer zunehmenden (progressiven) und/oder sprunghaften Änderung von Atmung, Hautdurchblutung, unwillkürlichen Bewegungszuständen, Hautfeuchtigkeit oder einer Kombination dieser Merkmale verbunden und zudem bei jedem Patienten bei Wiederholung im Wesentlichen gleich sind. Die erfindungsgemäße Signalvorrichtung erlaubt die individuelle Erfassung der wichtigsten vegetativen Schmerz-Stressreaktionen und damit eine zuverlässige Signalisierung eines Katheterisierungsbedarfs bei der Entstehung von Blasenhochdruck.

Vorzugsweise weist eine erfindungsgemäße Signalvorrichtung Stellmittel auf zum manuellen Einstellen einer Empfindlichkeit der Sensoren. Die Signalvorrichtung erlaubt so die individuelle Anpassung an unterschiedlich ausgeprägte Merkmale der Schmerz-Stressreaktion.

Gemäß der vorliegenden Erfindung weist die Signalvorrichtung ein Kontrollmittel auf zum manuellen Validieren des Signals. Die Signalvorrichtung ermöglicht so eine einfache Dokumentation und darauf basierende Justierung der Schwelle zum Erkennen der zunehmenden (progressiven) und/oder sprunghaften Zustandsänderung.

Als Stell- und Kontrollmittel kommen einerseits mechanische Dreh- oder Schieberegler und Taster in Betracht, die insbesondere an dem Datenverarbeitungsgerät ausgebildet sein können. Alternativ können virtuelle Taster solcher Elemente auf einem Bildschirm dargestellt werden, der in das Datenverarbeitungsgerät integriert oder mit diesem wiederum vorzugsweise über Funk verbunden - beispielsweise in einem Smartphone integriert - sein kann. Vorzugsweise weist eine solche erfindungsgemäße Signalvorrichtung ein Expertensystem auf zum automatischen Kalibrieren eines Schwellwerts für die zunehmende (progressive) und/oder sprunghafte Änderung anhand manueller Validierungen von Signalen mittels des Kontrollmittels. Die Signalvorrichtung bildet so ein in Bezug auf Schwelle zum Erkennen der zunehmenden (progressiven) und/oder sprunghaften Zustandsänderung kontinuierlich lernendes und sich selbst justierendes System.

Vorzugsweise weist eine erfindungsgemäße Signalvorrichtung weitere Sensoren auf für die Hautdurchblutung und -feuchtigkeit, für die Herzfrequenz und/oder für die elektrische Aktivität des Gehirns der Person. Die Signalvorrichtung erhält dann weitere Informationen zum Zustand der Person, die zum Erkennen der zunehmenden (progressiven) und/oder sprunghaften Zustandsänderung relevant sein können.

Zur Messung der Hautdurchblutung und arteriellen Sauerstoffsättigung kommen insbesondere Sensoren zur Reflexions- oder Transmissionsphotoplethysmographie (PPG, auch Pulsoximetrie) zum Einsatz, zur Messung der Hautfeuchtigkeit Sensoren für die elektrodermale Aktivität (galvanic skin response, GSR) und zur Messung der elektrischen Hirnaktivität herkömmliche EEG-Sensoren.

Vorzugsweise weist eine erfindungsgemäße Signalvorrichtung eine Echtzeituhr auf. In einer solchen Signalvorrichtung liefert die Echtzeituhr nicht nur einen für die Bestimmung des zeitlichen Abstands zu vorhergehenden Messungen nutzbaren regelmäßigen Takt, sondern bietet darüber hinaus auch die Möglichkeit einer Dokumentation der Messungen mit absoluten Zeitstempeln.

Vorzugsweise weist eine erfindungsgemäße Signalvorrichtung ein drahtlos mit dem Datenverarbeitungsgerät verbundenes - beispielsweise in einem Smartphone integriertes - Signalelement auf zum Erzeugen des Signals. Alternativ kann die erfindungsgemäße Signalvorrichtung auf ein in dem Datenverarbeitungsgerät integriertes Signalelement zugreifen. Als Signalelement kommen insbesondere vibrierende Elemente, Leuchtmittel, auch Bildschirme und Lautsprecher zum Einsatz

Vorzugsweise weist eine erfindungsgemäße Signalvorrichtung ein mit dem Datenverarbeitungsgerät verbundenes Steuerelement auf. Das Datenverarbeitungsgerät der Signalvorrichtung kann dann ohne Steuerelemente gestaltet und an einem vor unbefugtem oder unbeabsichtigtem Zugriff sowie vor anderen äußeren Einflüssen sicheren Ort untergebracht werden. Das Steuerelement ist vorzugsweise drahtlos mit dem Datenverarbeitungsgerät verbunden. Alternativ kommt auch eine kabelgebundene Lösung in Betracht.

Vorzugsweise ist in einer solchen erfindungsgemäßen Signalvorrichtung das Steuerelement in einem Smartphone implementiert. Beispielsweise kann das Steuerelement eine Softwareapplikation sein oder eine von dem Datenverarbeitungsgerät bereitgestellte Webseite, die in einem Browser des Smartphones aufgerufen wird. Alternativ kann das Steuerelement auch als Softwareapplikation oder Webseite auf einem gegebenen Falls nur vorübergehend durch Kabel mit dem Datenverarbeitungsgerät verbundenen Personal Computer (PC) implementiert sein. Weiter alternativ kann das Steuerelement eine Serverapplikation auf einem Server des Herstellers der Signalvorrichtung sein, mit dem sich das Datenverarbeitungsgerät selbststätig oder auf Anforderung über ein GSM-Modul oder über WLAN verbindet.

### Ausführungsbeispiel

Die Erfindung wird nachfolgend anhand eines Ausführungsbeispiels erläutert. Die in der Zeichnungsfigur gezeigte erfindungsgemäße Signalvorrichtung 1 besteht aus einem Datenverarbeitungsgerät 2, drei Sensoren 3 und einer auf einem Smartphone 4 installierten aber nicht weiter dargestellten Anwendungsapplikation.

Die Sensoren 3 sind eine Sensormatte zur Messung der elektrischen Herztätigkeit (EKG) und der Atmung einer nicht dargestellten Person, ein Reflexionsphotoplethysmographie-Sensor zur Messung der Hautdurchblutung, ein Bewegungssensor zur Messung willkürlicher und unwillkürlicher Bewegungen und ein GSR-Sensor zur Messung der Hautfeuchtigkeit. Die Sensoren 3 weisen jeweils ein Bluetooth-Funkmodul auf.

Das Datenverarbeitungsgerät 2 ist ein handelsüblicher Kleinstcomputer mit Prozessor, Arbeitsspeicher, Echtzeituhr und Bluetooth-Funkmodul, der mit einer Software dazu eingerichtet ist, von den Sensoren 3 aufgenommene Daten zu einem Zustand der Person drahtlos zu empfangen und bei einer zunehmenden (progressiven) und/oder sprunghaften Änderung des Zustands ein Signal zu erzeugen

Zur Anwendung der Signalvorrichtung 1 werden zunächst die Sensoren 3 an der Person an Körperstellen appliziert, die den oft körperbehinderten Anwender nicht weiter in seiner Beweglichkeit einschränken. Da erfahrungsgemäß Schmerz-Stressreaktionen bei neurologisch geschädigten Personen nicht immer gleichmäßig im Körper auftreten, werden die Sensoren 3 so wenig belastend wie möglich an Prädelektionsstellen appliziert. Dann wird das Datenverarbeitungsgerät 2 über Bluetooth mit dem Smartphone 4 verbunden und mittels der Anwendungsapplikation konfiguriert.

Das Datenverarbeitungsgerät 2 erkennt die in der Nähe befindlichen Sensoren 3, stellt zu diesen eine Datenverbindung über Bluetooth her und nimmt die Startkonfiguration der Messung vor. Das Datenverarbeitungsgerät 2 definiert hierzu einen Messrhythmus, teilt hierzu den Abstand zwischen zwei Messungen in Zeitfenster und weist jedem der Sensoren 3 eines der Zeitfenster zu. Von diesem Zeitpunkt senden die Sensoren 3 in dem Messrhythmus ihren jeweiligen Messwert zum Zustand der Person in dem zugewiesenen Zeitfenster an das Datenverarbeitungsgerät 2.

Das Datenverarbeitungsgerät 2 analysiert die Zeitreihen der Messungen und erkennt selbständig mit einer zunächst sehr hohen Sensibilität eine zunehmende (progressive) und/oder sprunghafte Änderung des Zustands der Person. Sobald das Datenverarbeitungsgerät 2 eine zunehmende (progressive) und/oder sprunghafte Änderung erkennt, erzeugt die Anwendungsapplikation durch Vibration, einen Signalton und ein Aufleuchten einer LED ein taktiles, akustisches und visuelles Signal für einen Katheterisierungsbedarf und fordert den Nutzer auf, den Katheterisierungsbedarf durch Betätigen eines auf dem Bildschirm dargestellten Tasters zu validieren, also den Katheterisierungsbedarf zu bestätigen oder abzulehnen.

Mit jedem validierten Signal lernt ein in dem Datenverarbeitungsgerät 2 implementiertes Expertensystem, welche zunehmende (progressive) und/oder sprunghafte Änderung der gemessenen Werte einen tatsächlichen Katheterisierungsbedarf bedeutet. Die Zahl der Fehlsignale sinkt etwa exponentiell mit der Zahl der Signale. Durch Analyse der Messreihen ermittelt das Datenverarbeitungsgerät 2 auch die tatsächlich erforderliche Messempfindlichkeit der Sensoren 3 sowie den tatsächlich benötigten Messrhythmus und passt beides gegebenen Falls automatisch an.

Über die Anwendungsapplikation kann der Nutzer darüber hinaus jederzeit die über virtuelle Schiebeelemente die Messempfindlichkeit der Signalvorrichtung 1 insgesamt sowie für jeden einzelnen Sensor 3 erhöhen oder vermindern sowie den Messrhythmus verlängern oder verkürzen.

Die Anwendungsapplikation verbindet sich regelmäßig, wenn das Smartphone 4 eine Datenverbindung zur Verfügung stellt, über das Internet 5 mit einem Server 6 des Herstellers und überträgt an diesen anonymisierte Betriebsdaten der Signalvorrichtung 1. Anhand dieser Daten wird sowohl die Anwendungsapplikation als auch die Software des Datenverarbeitungsgeräts 2 kontinuierlich verbessert.

### In den Figuren sind

- 1: Signalvorrichtung,
- 2: Datenverarbeitungsgerät
- 3: Sensor
- 4: Smartphone
- 5: Internet
- 6: Server

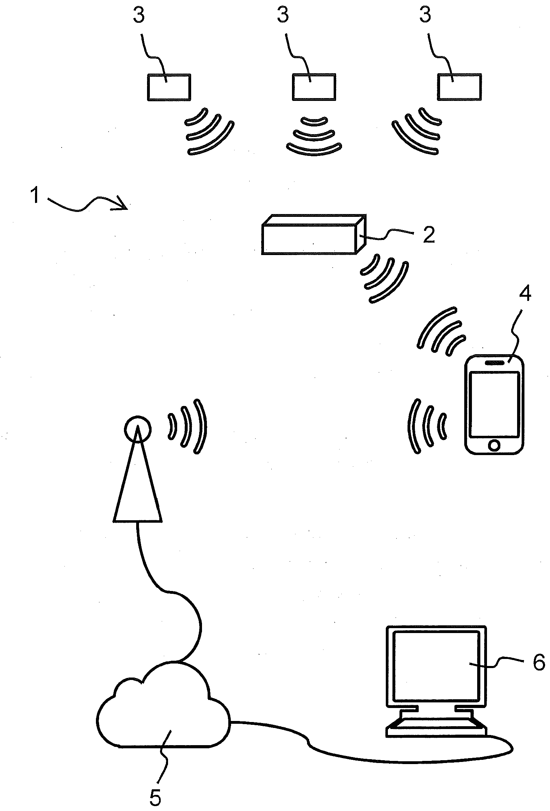

## Patentansprüche

1. Verwendung einer Signalvorrichtung (1) zur Ermittlung eines individuellen Katheterisierungsbedarfs einer Person vor Entstehen eines Blasenhochdrucks, wobei die Signalvorrichtung (1) ein Datenverarbeitungsgerät (2) aufweist und Sensoren (3) für Herzfrequenz, Atmung und Bewegung einer Person, die jeweils Funkverbindungsmittel zur drahtlosen Verbindung mit dem Datenverarbeitungsgerät (2) aufweisen, wobei das Datenverarbeitungsgerät (2) dazu eingerichtet ist, von den Sensoren (3) aufgenommene Daten zu einem physiologischen Zustand der Person drahtlos zu empfangen und bei einer zunehmenden und/oder sprunghaften Änderung des Zustands ein akustisches, visuelles und/oder taktiles Signal zu erzeugen und wobei die Signalvorrichtung (1) ein Kontrollmittel zum manuellen Validieren des Signals aufweist.

2. Verwendung nach dem vorgenannten Anspruch, wobei die Signalvorrichtung (1) ***gekennzeichnet ist durch*** Stellmittel zum manuellen Einstellen einer Empfindlichkeit der Sensoren (3).

3. Verwendung nach einem der vorgenannten Ansprüche, wobei die Signalvorrichtung (1) ***gekennzeichnet ist durch*** ein Expertensystem zum automatischen Kalibrieren eines Schwellwerts für die zunehmende und/oder sprunghafte Änderung anhand manueller Validierungen von Signalen mittels des Kontrollmittels.

4. Verwendung nach einem der vorgenannten Ansprüche, wobei die Signalvorrichtung (1) ***gekennzeichnet ist durch*** weitere Sensoren für die Hautdurchblutung und -feuchtigkeit, für die Herzfrequenz und/oder für die elektrische Aktivität des Gehirns der Person.

5. Verwendung nach einem der vorgenannten Ansprüche, wobei die Signalvorrichtung (1) ***gekennzeichnet ist durch*** eine Echtzeituhr.

6. Verwendung nach einem der vorgenannten Ansprüche, wobei die Signalvorrichtung (1) ***gekennzeichnet ist durch*** ein drahtlos mit dem Datenverarbeitungsgerät (2) verbundenes Signalelement zum Erzeugen des Signals.

7. Verwendung nach einem der vorgenannten Ansprüche, wobei die Signalvorrichtung (1) ***gekennzeichnet ist durch*** ein mit dem Datenverarbeitungsgerät (2) verbundenes Steuerelement.

8. Verwendung nach dem vorgenannten Anspruch, wobei die Signalvorrichtung (1) ***dadurch gekennzeichnet ist, dass** das* Steuerelement in einem Smartphone (4) implementiert ist.

## Claims

1. Use of a signalling device (1) for determining an individual need for catheterization of a person before high bladder pressure occurs, wherein the signalling device (1) has a data processing device (2) and sensors (3) for heart rate, breathing and movement of a person which respectively have radio communication means for wireless connection to the data processing device (2), wherein the data processing device (2) is configured to wirelessly receive data captured by the sensors (3) regarding a physiological condition of the person and to generate an acoustic, visual and/or tactile signal in the event of an increasing and/or rapid change in the condition, and wherein the signalling device (1) has a control means for manually validating the signal.

2. Use as claimed in the preceding claim, wherein the signalling device (1) is **characterized by** adjustment means for manually adjusting a sensitivity of the sensors (3).

3. Use as claimed in one of the preceding claims, wherein the signalling device (1) is **characterized by** an expert system for the automatic calibration of a threshold value for the increasing and/or rapid change with the aid of manual validations of signals by means of the control means.

4. Use as claimed in one of the preceding claims, wherein the signalling device (1) is **characterized by** additional sensors for skin blood circulation and skin moisture, for the heart rate and/or for the electrical activity of the brain of the person.

5. Use as claimed in one of the preceding claims, wherein the signalling device (1) is **characterized by** a real time clock.

6. Use as claimed in one of the preceding claims, wherein the signalling device (1) is **characterized by** a signalling element for generating the signal, which is wirelessly connected to the data processing device (2).

7. Use as claimed in one of the preceding claims, wherein the signalling device (1) is **characterized by** a control element which is connected to the data processing device (2) .

8. Use as claimed in one of the preceding claims, wherein the signalling device (1) is **characterized in that** the control element is implemented in a smartphone (4).

## Revendications

1. Utilisation d'un dispositif de signalisation (1) pour la détermination d'un besoin individuel de cathétérisation d'une personne avant l'apparition d'une haute pression de vessie, le dispositif de signalisation (1) présentant un appareil de traitement de données (2) et des capteurs (3) pour la fréquence cardiaque, la respiration et le mouvement d'une personne, qui présentent respectivement des moyens de liaison radio pour la connexion sans fil avec l'appareil de traitement des données (2), l'appareil de traitement de données (2) étant conçu pour recevoir des données enregistrées par les capteurs (3) concernant un état physiologique de la personne et, dans le cas d'une modification croissante et/ou par à-coups de l'état, générer un signal acoustique, visuel et/ou tactile et le dispositif de signalisation (1) présentant un moyen de contrôle pour la validation manuelle du signal.

2. Utilisation selon la revendication précédente, dans laquelle le dispositif de signalisation (1) est **caractérisé par** des moyens de réglage pour le réglage manuel d'une sensibilité des capteurs (3).

3. Utilisation selon une des revendications précédentes, dans laquelle le dispositif de signalisation (1) est **caractérisé par** un système expert d'étalonnage automatique d'une valeur seuil pour la modification croissante et/par à-coups sur la base de validations manuelles de signaux au moyen du moyen de contrôle.

4. Utilisation selon une des revendications précédentes, dans laquelle le dispositif de signalisation (1) est **caractérisé par** d'autres capteurs pour l'irrigation sanguine et l'humidité de la peau, pour la fréquence cardiaque et/ou pour l'activité électrique du cerveau de la personne.

5. Utilisation selon une des revendications précédentes, dans laquelle le dispositif de signalisation (1) est **caractérisé par** une horloge en temps réel.

6. Utilisation selon une des revendications précédentes, dans laquelle le dispositif de signalisation (1) est **caractérisé par** un élément de signalisation connecté sans fil à l'appareil de traitement de données (2) pour la génération du signal.

7. Utilisation selon une des revendications précédentes, dans laquelle le dispositif de signalisation (1) est **caractérisé par** un élément de commande connecté à l'appareil de traitement de données (2).

8. Utilisation selon une des revendications précédentes, dans laquelle le dispositif de signalisation (1) est **caractérisé en ce que** l'élément de commande est mis en œuvre dans un Smartphone (4).
